(19) Europäisches Patentamt
European Patent Office
Office européen des brevets

(11) Veröffentlichungsnummer: **0 158 157**
**A1**

(12) # EUROPÄISCHE PATENTANMELDUNG

(21) Anmeldenummer: 85103022.1

(22) Anmeldetag: 15.03.85

(51) Int. Cl.⁴: **A 61 K 31/40**
A 61 K 31/435, C 07 D 209/52
C 07 D 209/54, C 07 D 209/42

(30) Priorität: 23.03.84 DE 3410732

(43) Veröffentlichungstag der Anmeldung: 16.10.85 Patentblatt 85/42

(84) Benannte Vertragsstaaten: AT BE CH DE FR GB IT LI LU NL SE

(71) Anmelder: **HOECHST AKTIENGESELLSCHAFT**
**Postfach 80 03 20**
**D-6230 Frankfurt am Main 80(DE)**

(72) Erfinder: **Urbach, Hansjörg, Dr.**
**Le Lavandoustrasse 41**
**D-6242 Kronberg/Taunus(DE)**

(72) Erfinder: **Henning, Rainer, Dr.**
**Rotenhofstrasse 31**
**D-6234 Hattersheim am Main(DE)**

(72) Erfinder: **Geiger, Rolf, Prof. Dr.**
**Heinrich-Bleicher-Strasse 33**
**D-6000 Frankfurt am Main 50(DE)**

(72) Erfinder: **Teetz, Volker, Dr.**
**An der Tann 20**
**D-6238 Hofheim am Taunus(DE)**

(54) **Methode zur Behandlung des Glaukoms.**

(57) Die Erfindung betrifft eine Methode zur Behandlung des Glaukoms und/oder der Herabsetzung des Augeninnendrucks bei Säugern durch topische oder systemische Anwendung von Verbindungen der Formel I

$$R^3OOC-\overset{*}{C}H(R^4)-N(R^5)-C(=O)-\overset{*}{C}H(R^1)-NH-\overset{*}{C}H(COOR^2)-(CH_2)_n-R \qquad (I)$$

in der n=1 oder 2 ist, R, $R^1$, $R^2$ und $R^3$ gleich oder verschieden sind und jeweils Wasserstoff oder einen organischen Rest bedeuten, und $R^4$ und $R^5$ zusammen mit den sie tragenden Atomen ein mono-, bi- oder tricyclisches heterocyclisches Ringsystem bilden. Die Erfindung betrifft weiterhin Verbindungen der Formel I sowie diese enthaltende Mittel zur Anwendung bei der Behandlung der oben genannten Krankheiten.

EP 0 158 157 A1

Croydon Printing Company Ltd

Methode zur Behandlung des Glaukoms

Die Erfindung betrifft eine Methode zur Behandlung des Glaukoms und/oder der Herabsetzung des Augeninnendrucks bei Säugern, vorzugsweise beim Menschen durch topische oder systemische Anwendung von Angiotensin-Converting-Enzyme-Inhibitoren der Formel I

$$R^3OOC - \overset{*}{\underset{R^4}{CH}} - \underset{R^5}{N} - \underset{O}{\overset{\|}{C}} - \overset{*}{\underset{R^1}{CH}} - NH - \overset{*}{\underset{COOR^2}{CH}} - (CH_2)_n-R \qquad (I)$$

in welcher

n= 1 oder 2 ist,

R= Wasserstoff,

einen gegebenenfalls substituierten aliphatischen Rest mit 1 - 8 C-Atomen,

einen gegebenenfalls substituierten alicyclischen Rest mit 3 - 9 C-Atomen,

einen gegebenenfalls substituierten aromatischen Rest mit 6 - 12 C-Atomen,

einen gegebenenfalls substituierten araliphatischen Rest mit 7 - 14 C-Atomen,

einen gegebenenfalls substituierten alicyclisch-aliphatischen Rest mit 7 - 14 C-Atomen,

einen Rest $OR^a$ oder $SR^a$, worin

$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,

$R^1$ Wasserstoff,

einen gegebenenfalls substituierten aliphatischen Rest mit 1 - 6 C-Atomen,

einen gegebenenfalls substituierten alicyclischen Rest mit 3 - 9 C-Atomen,

einen gegebenenfalls substituierten alicyclisch-aliphatischen Rest mit 4 - 13 C-Atomen,
einen gegebenenfalls substituierten aromatischen Rest mit 6 - 12 C-Atomen,
einen gegebenenfalls substituierten araliphatischen Rest mit 7 - 16 C-Atomen,
einen gegebenenfalls substituierten heteroaromatischen Rest mit 5 - 12 Ringatomen oder
die erforderlichenfalls geschützte Seitenkette einer natürlich vorkommenden α-Aminosäure bedeuten,

$R^2$ und $R^3$ gleich oder verschieden sind und Wasserstoff,
einen gegebenenfalls substituierten aliphatischen Rest mit 1 - 6 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 16 C-Atomen bedeuten und

$R^4$ und $R^5$ zusammen mit den sie tragenden Atomen ein mono-, bi-, oder tricyclisches heterocyclisches Ringsystem mit 5 bis 15 C-Atomen bilden sowie deren physiologisch verträglichen Salzen.

Als solche Ringsysteme kommen insbesondere jene aus der folgenden Gruppe in Betracht:

Tetrahydroisochinolin (A); Decahydroisochinolin (B); Octahydroindol (C); Octahydrocyclopenta [b]pyrrol (D); 2-Aza-bicyclo[2.2.2]octan (E); 2-Azabicyclo[2.2.1]heptan (F); 2-Azaspiro[4.5]decan (G); 2-Azaspiro[4.4]nonan (H); Spiro[(bicyclo[2.2.1]heptan)-2,3-pyrrolidin] (I); Spiro-[(bicyclo[2.2.2]octan)-2,3-pyrrolidin] (J); 2-Azatricyclo[4,3,0,$1^{6,9}$]decan (K); Decahydrocyclohepta[b]pyrrol (L); Octahydroisoindol (M); Octahydrocyclopenta[c]pyrrol

(N); 2,3,3a,4,5,7a-Hexahydroindol(O); 2-Azabicyclo[3.1.0]-hexan (P); die alle gegebenenfalls substituiert sein können. Bevorzugt sind jedoch die unsubstituierten Systeme.

Bei den Verbindungen, die mehrere chirale Atome besitzen, kommen alle möglichen Diastereomere als Racemate oder Enantiomere, oder Gemische verschiedener Diastereomere in Betracht.

Die in Betracht kommenden cyclischen Aminosäureester weisen die folgenden Strukturformeln auf.

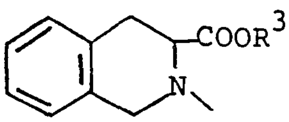
A

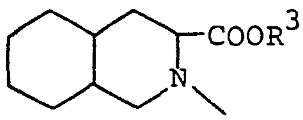
B

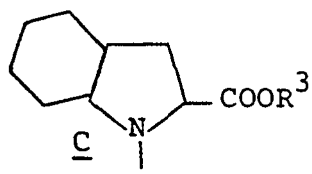
C

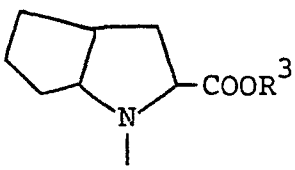
D

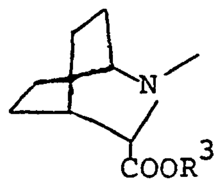
E

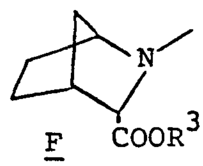
F

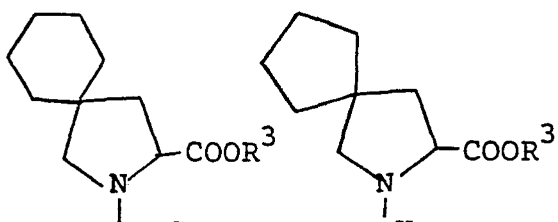
G H

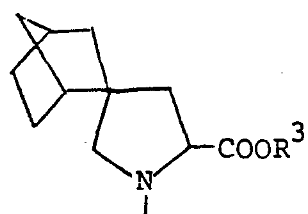
I

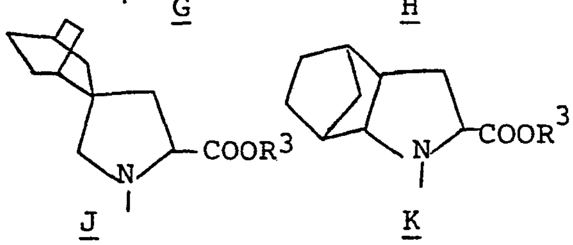
J K

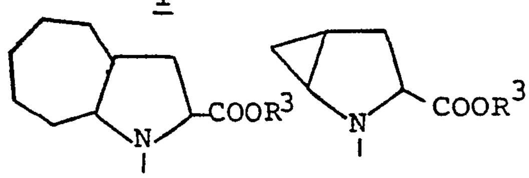
L P

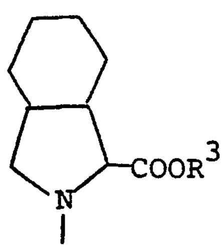
M

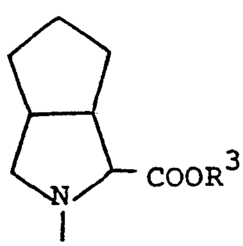
N

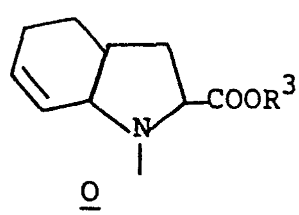
O

Eine bevorzugte Ausführungsform ist dadurch gekennzeichnet, daß Verbindungen der Formel I angewendet werden, in der

n= 1 oder 2 ist

R Wasserstoff,

Alkyl mit 1 - 8 C-Atomen,

Alkenyl mit 2 - 6 C-Atomen,

Cycloalkyl mit 3 - 9 C-Atomen,

Aryl mit 6 - 12 C-Atomen,

das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-Alkylamino, Methylendioxy, Carboxy, Cyano und/oder Sulfamoyl mono-, di- oder trisubstituiert sein kann,

Alkoxy mit 1 - 4 C-Atomen,

Aryloxy mit 6 - 12 C-Atomen,

das wie oben bei Aryl beschrieben substituiert sein kann,

mono- bzw. bicyclisches Heteroaryloxy mit 5 - 7 bzw. 8 - 10 Ringatomen, wovon 1 bis 2 Ringatome Schwefel- oder Sauerstoffatome und/oder 1 bis 4 Ringatome Stickstoff darstellen,

das wie oben bei Aryl beschrieben substituiert sein kann,

Amino-($C_1$-$C_4$)-alkyl,

($C_1$-$C_4$)-Alkanoylamino-($C_1$-$C_4$)alkyl,

($C_7$-$C_{13}$)-Aroylamino-($C_1$-$C_4$)-alkyl,

($C_1$-$C_4$)-Alkoxy-carbonylamino-($C_1$-$C_4$)-alkyl,

($C_6$-$C_{12}$)-Aryl-($C_1$-$C_4$)-alkoxycarbonylamino-($C_1$-$C_4$)-alkyl,

($C_6$-$C_{12}$)-Aryl-($C_1$-$C_4$)-alkylamino-($C_1$-$C_4$)-alkyl,

($C_1$-$C_4$)-Alkylamino-($C_1$-$C_4$)-alkyl,

Di-($C_1$-$C_4$)-alkylamino-($C_1$-$C_4$)-alkyl,

Guanidino-($C_1$-$C_4$)-alkyl,

Imidazolyl, Indolyl,

($C_1$-$C_4$)-Alkylthio,

($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,

$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann,
($C_6$-$C_{12}$ )-Aryl-($C_1$-$C_4$)-alkyl oder ($C_7$-$C_{13}$)-Aroyl-($C_1$ oder $C_2$ )alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,

das wie das vorstehende Aryl substituiert sein kann oder

die gegebenenfalls geschützte Seitenkette einer natürlich vorkommenden α-Aminosäure $R^1$-CH($NH_2$)-COOH bedeuten,

$R^2$ und $R^3$ gleich oder verschieden sind und

Wasserstoff,

Alkyl mit 1 - 6 C-Atomen,

Alkenyl mit 2 - 6 C-Atomen,

Di-($C_1$-$C_4$)-alkylamino-($C_1$-$C_4$)-alkyl,

($C_1$-$C_5$)-Alkanoyloxy-($C_1$-$C_4$)-alkyl,

($C_1$-$C_6$)-Alkoxy-carbonyloxy-($C_1$-$C_4$)-alkyl,

($C_7$-$C_{13}$)-Aroyloxy-($C_1$-$C_4$)-alkyl,

($C_6$-$C_{12}$)-Aryloxycarbonyloxy($C_1$-$C_4$)-alkyl,

Aryl mit 6 - 12 C-Atomen,

($C_6$-$C_{12}$)-Aryl-($C_1$-$C_4$)-alkyl,

($C_3$-$C_9$)-Cycloalkyl oder

($C_3$-$C_9$)-Cycloalkyl-($C_1$-$C_4$)-alkyl

bedeuten und

$R^4$ und $R^5$ die oben angegebene Bedeutung haben.

Besonders bevorzugt ist eine Ausführungsform, die dadurch gekennzeichnet ist, daß Verbindungen der Formel I angewendet werden, in der

n= 1 oder 2 ist,

R ($C_1$-$C_6$)-Alkyl, ($C_2$-$C_6$)-Alkenyl, ($C_3$-$C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl, das durch ($C_1$-$C_4$)-Alkyl, ($C_1$-$C_4$)-Alkoxy, Hydroxy, Halogen, Nitro, Amino, ($C_1$-$C_4$)-Alkylamino, Di-($C_1$-$C_4$)-alkylamino und/oder Methylendioxy mono-, di- oder trisubstituiert sein kann, oder 3-Indolyl, insbesondere Methyl, Ethyl, Cyclohexyl, tert.Butoxycarbonylamino-

$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-Alkyl oder $(C_7-C_{13})$-Aroyl-$(C_1-C_2)$-alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, $(C_1-C_6)$-Alkyl, $(C_2-C_6)$-Alkenyl oder $(C_6-C_{12})$-Aryl-$(C_1-C_4)$-alkyl, insbesondere aber Wasserstoff, $(C_1-C_4)$-Alkyl oder Benzyl bedeuten und

$R^4$ und $R^5$ die oben angegebene Bedeutung haben.

Insbesondere bevorzugt ist die Anwendung von Verbindungen der Formel I, in welcher n= 2 ist, R= Phenyl, $R^1$= Methyl,

$R^2$ und $R^3$ gleiche oder verschiedene ($C_1$-$C_6$)-Alkylreste oder ($C_7$-$C_{10}$)-Aralkylreste wie Benzyl oder Nitrobenzyl bedeuten und $R^4$ und $R^5$ zusammen für einen Rest der Formel steht,

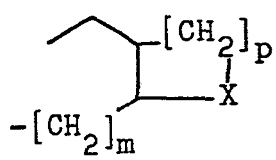

worin m= 0 oder 1, p= 0, 1 oder 2 und X= $-CH_2-$, $-CH_2-CH_2-$ oder -CH=CH- bedeuten, wobei ein mit X gebildeter 6-Ring auch ein Benzolring sein kann.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z.B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z.B. geschütztes Ser, Thr, Asp, Asn, Glu, Gln, Arg, Lys, Hyl, Cys, Orn, Cit, Tyr, Trp, His oder Hyp, sind als Schutzgruppen in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder $(C_1-C_6)$-Alkanoyl bevorzugt. Als O-Schutzgruppen für Tyrosin kommen bevorzugt $(C_1-C_6)$-Alkyl, insbesondere Methyl oder Ethyl in Frage.

Besonders vorteilhaft können die folgenden Verbindungen nach der erfindungsgemäßen Methode angewendet werden:

N-(1-S-Carbethoxy-3-phenyl-propyl)-S-alanyl-S-1,2,3,4-tetrahydroisochinolin-3-carbonsäure
N-(1-S-Carbethoxy-3-cyclohexyl-propyl)-S-alanyl-S-1,2,3,4-tetrahydroisochinolin-3-carbonsäure
N-(1-S-Carbethoxy-3-phenyl-propyl)-S-lysyl-S-1,2,3,4-tetrahydroisochinolin-3-carbonsäure
N-(1-S-Carbethoxy-3-phenyl-propyl)-O-ethyl-S-tyrosyl-S-1,2,3,4-tetrahydroisochinolin-3-carbonsäure
N-(1-S-Carbethoxy-3-phenyl-propyl)-S-alanyl-3S-decahydroisochinolin-3-carbonsäure
N-(1-S-Carbethoxy-3-phenyl-propyl)-S-alanyl-(2S,3aS,7aS)-octahydroindol-2-carbonsäure
N-(1-S-Carbethoxy-3-cyclohexyl-propyl)-S-alanyl-(2S,3aS,7aS)-octahydroindol-2-carbonsäure
N-(1-S-Carbethoxy-3-phenyl-propyl)-S-lysyl-(2S,3aS,7aS)-octahydroindol-2-carbonsäure
N-(1-S-Carbethoxy-n-butyl)-S-alanyl-(2S,3aS,7aS)-octahydroindol-2-carbonsäure

N-(1-S-Carbethoxy-3-cyclohexyl-propyl)-S-lysyl-(2S,3aS,7aS)-octahydroindol-2-carbonsäure

N-(1-S-Carbethoxy-3-phenyl-propyl)-O-methyl-S-tyrosyl-(2S,3aS,7aS)-octahydroindol-2-carbonsäure

N-(1-S-Carbethoxy-3-phenyl-propyl)-O-ethyl-S-tyrosyl-(2S,3aS,7aS)-octahydroindol-2-carbonsäure

N-(1-S-Carbethoxy-3-(3,4-dimethylphenyl-propyl)-S-alanyl-(2S,3aS,7aS)-octahydroindol-2-carbonsäure

N-[1-S-Carbethoxy-3-(4-fluorphenyl)-propyl]-S-alanyl-(2S,3aS,7aS)-octahydroindol-2-carbonsäure

N-[1-S-Carbethoxy-3-(4-methoxyphenyl)-propyl]-S-alanyl-(2S,3aS,7aS)-octahydroindol-2-carbonsäure

N-[1-S-Carbethoxy-3-(3,4-dimethoxyphenyl)-propyl]-S-alanyl-(2S,3aS,7aS)-octahydroindol-2-carbonsäure

N-(1-S-Carbethoxy-3-cyclopentylpropyl)-S-alanyl-(2S,3aS,7aS)-octahydroindol-2-carbonsäure

N-(1-S-Carbethoxy-3-phenyl-propyl)-S-alanyl-(2S,3aR,7aS)-octahydroindol-2-carbonsäure

N-(1-S-Carbethoxy-3-cyclohexyl-propyl)-S-alanyl-(2S,3aR,7aS)-octahydroindol-2-carbonsäure

N-(1-S-Carbethoxy-3-phenyl-propyl)-S-lysyl-(2S,3aR,7aS)-octahydroindol-2-carbonsäure

N-(1-S-Carbethoxy-3-cyclohexyl-propyl)-S-lysyl-(2S,3aR,7aS)-octahydroindol-2-carbonsäure

N-(1-S-Carbethoxy-3-phenyl-propyl)-O-ethyl-S-tyrosyl-(2S,3aR,7aS)-octahydroindol-2-carbonsäure

N-(1-S-Carbethoxy-3-phenyl-propyl)-S-alanyl-(2S,3aR,7aR)-octahydroindol-2-carbonsäure

N-(1-S-Carbethoxy-3-phenyl-propyl)-S-lysyl-(2S,3aR,7aR)-octahydroindol-2-carbonsäure

N-(1-S-Carbethoxy-3-cyclohexyl-propyl)-S-alanyl-(2S,3aR,7aR)-octahydroindol-2-carbonsäure

N-(1-S-Carbethoxy-3-cyclohexyl-propyl)-O-ethyl-S-tyrosyl-(2S,3aR,7aR)-octahydroindol-2-carbonsäure

N-(1-S-Carbethoxy-3-phenyl-propyl)-S-alanyl-(2S,3aS,7aR)-octahydroindol-2-carbonsäure

N-(1-S-Carbethoxy-3-phenyl-propyl)-O-ethyl-S-tyrosyl-(2S,3aS,7aS)-octahydroindol-2-carbonsäure

N-(1-S-Carbethoxy-3-(3,4-dimethylphenyl-propyl)-S-alanyl-(2S,3aS,7aS)-octahydroindol-2-carbonsäure

N-[1-S-Carbethoxy-3-(4-fluorphenyl)-propyl]-S-alanyl-(2S,3aS,7aS)-octahydroindol-2-carbonsäure

N-[1-S-Carbethoxy-3-(4-methoxyphenyl)-propyl]-S-alanyl-(2S,3aS,7aS)-octahydroindol-2-carbonsäure

N-[1-S-Carbethoxy-3-(3,4-dimethoxyphenyl)-propyl]-S-alanyl-(2S,3aS,7aS)-octahydroindol-2-carbonsäure

N-(1-S-Carbethoxy-3-cyclopentylpropyl)-S-alanyl-(2S,3aS,7aS)-octahydroindol-2-carbonsäure

N-(1-S-Carbethoxy-3-phenyl-propyl)-S-alanyl-cis-endo-2-azabicyclo[3.3.0]octan-3-S-carbonsäure

N-(1-S-Carbethoxy-3-phenyl-propyl)-S-lysyl-cis-endo-2-azabicyclo[3.3.0]octan-3-S-carbonsäure

N-(1-S-Carbethoxy-3-cyclohexyl-propyl)-S-alanyl-cis-endo-2-azabicyclo[3.3.0]octan-3-S-carbonsäure

N-(1-S-Carboxy-3-cyclohexyl-propyl)-S-alanyl-cis-endo-2-azabicyclo[3.3.0]octan-3-S-carbonsäure

N-(1-S-Carbethoxy-butyl)-S-alanyl-cis-endo-2-azabicyclo-[3.3.0]octan-3-S-carbonsäure

N-(1-S-Carbethoxy-3-phenyl-propyl)-O-methyl-S-tyrosyl-cis-endo-2-azabicyclo[3.3.0]octan-3-S-carbonsäure

N-(1-S-Carbethoxy-3-phenyl-propyl)-O-ethyl-S-tyrosyl-cis-endo-2-azabicyclo[3.3.0]octan-3-S-carbonsäure

N-(1-S-Carbethoxy-3-(4-fluorphenyl-propyl)-S-alanyl-cis-endo-azabicyclo[3.3.0]octan-3-S-carbonsäure

N-(1-S-Carbethoxy-3-(4-methoxyphenyl-propyl)-S-alanyl-cis-endo-2-azabicyclo[3.3.0]octan-3-S-carbonsäure

N-(1-S-Carbethoxy-3-(3,4-dimethoxyphenyl-propyl)-S-alanyl-cis-endo-2-azabicyclo[3.3.0]octan-3-S-carbonsäure

N-(1-S-Carbethoxy-3-cyclopentyl-propyl)-S-alanyl-cis-endo-2-azabicyclo[3.3.0]octan-3-S-carbonsäure

N-(1-S-Carbethoxy-3-phenyl-propyl)-S-lysyl-(2S,3aR,6aS)-octahydrocyclopenta[b]pyrrol-2-carbonsäure

N-(1-S-Carbethoxy-3-cyclohexylpropyl)-S-lysyl-(2S,3aR,6aS)-octahydrocyclopenta[b]pyrrol-2-carbonsäure

N-(1-S-Carbethoxy-3-phenyl-propyl)-O-ethyl-S-tyrosyl-(2S,3aR,6aS)-octahydrocyclopenta[b]pyrrol-2-carbonsäure

N-(1-S-Carbethoxy-3-cyclopentyl-propyl)-S-alanyl-2-(2S,3aR,6aS)-octahydrocyclopenta[b]pyrrol-2-carbonsäure

N-(1-S-Carbethoxy-3-phenyl-propyl)-S-alanyl-(2S,3aR,6aS)-octahydrocyclopenta[b]pyrrol-2-carbonsäure

N-(1-S-Carbethoxy-3-phenyl-propyl)-S-alanyl-S-2-azabicyclo[2.2.2]octan-3-carbonsäure

N-(1-S-Carbethoxy-3-phenyl-propyl)-O-ethyl-S-tyrosyl-S-2-azabicyclo[2.2.2]octan-3-carbonsäure

N-(1-S-Carbethoxy-3-cyclohexyl-propyl)-S-alanyl-S-2-azabicyclo[2.2.2]octan-3-carbonsäure

N-(1-S-Carbethoxy-3-cyclohexyl-propyl)-S-2-azabicyclo-[2.2.2]octan-3-carbonsäure

N-(1-S-Carbethoxy-3-phenyl-propyl)-S-lysyl-S-2-azabicyclo-[2.2.2]octan-3-carbonsäure

N-(1-S-Carbethoxy-3-cyclopentylpropyl)-S-alanyl-S-2-azabicyclo[2.2.2]octan-3-carbonsäure

N-(1-S-Carbethoxy-3-phenyl-propyl)-S-alanyl-3S-exo-2-azabicyclo[2.2.1]-heptan-3-carbonsäure

N-(1-S-Carbethoxy-3-cyclohexylpropyl)-S-alanyl-3S-exo-2-azabicyclo[2.2.1]heptan-3-carbonsäure

N-(1-S-Carbethoxy-3-phenyl-propyl)-S-lysyl-3S-exo-2-azabicyclo[2.2.1]heptan-3-carbonsäure

N-(1-S-Carbethoxy-3-phenyl-propyl)-S-alanyl-3S-endo-2-azabicyclo[2.2.1]heptan-3-carbonsäure

N-(1-S-Carbethoxy-3-cyclohexylpropyl)-S-alanyl-3S-endo-2-azabicyclo[2.2.1]heptan-3-carbonsäure

N-(1-S-Carbethoxy-3-phenyl-propyl)-S-lysyl-3S-endo-2-azabicyclo[2.2.1]heptan-3-carbonsäure

N-(1-S-Carbethoxy-3-phenyl-propyl)-O-ethyl-S-tyrosyl-3S-endo-2-azabicyclo[2.2.1]heptan-3-carbonsäure

N-(1-S-Carbethoxy-3-phenyl-propyl)-S-alanyl-2-azaspiro-[4,5]decan-3-S-carbonsäure

N-(1-S-Carbethoxy-3-phenyl-propyl)-O-ethyl-S-tyrosyl-2-azaspiro[4,5]decan-3-S-carbonsäure

N-(1-S-Carbethoxy-3-phenyl-propyl)-S-lysyl-2-azaspiro-[4,5]decan-3-S-carbonsäure

N-(1-S-Carbethoxy-3-cyclohexylpropyl)-S-alanyl-2-azaspiro [4,5]decan-3-S-carbonsäure

N-(1-S-Carbethoxy-3-cyclohexylpropyl)-S-lysyl-2-azaspiro-[4,5]decan-3-S-carbonsäure

N-(1-S-Carbethoxy-3-phenyl-propyl)-S-alanyl-2-azaspiro-[4,4]nonan-3-S-carbonsäure

N-(1-S-Carbethoxy-3-phenyl-propyl)-O-ethyl-S-tyrosyl-2-azaspiro[4,4]nonan-3-S-carbonsäure

N-(1-S-Carbethoxy-3-phenyl-propyl)-S-lysyl-2-azaspiro-[4,4]nonan-3-S-carbonsäure

N-(1-S-Carbethoxy-3-cyclohexyl-propyl)-S-alanyl-2-aza-spiro[4,4]nonan-3-S-carbonsäure

N-(1-S-Carbethoxy-3-cyclopentyl-propyl)-S-alanyl-2-aza-spiro[4,4]nonan-3-S-carbonsäure

N-(1-S-Carbethoxy-3-cyclopentyl-propyl)-S-lysyl-2-aza-spiro[4,4]nonan-3-S-carbonsäure

N-(1-S-Carbethoxy-3-phenyl-propyl)-S-alanyl-spiro[bicyclo-[2.2.1]heptan-2,3'-pyrrolidin]-5'-S-carbonsäure

N-(1-S-Carbethoxy-3-phenyl-propyl)-O-ethyl-S-tyrosyl-spiro[bicyclo[2.2.1]heptan-2,3'-pyrrolidin]-5'-S-carbonsäure

N-(1-S-Carbethoxy-3-phenyl-propyl)-S-lysyl-spiro[bicyclo-[2.2.1]heptan-2,3'-pyrrolidin]-5'-S-carbonsäure

N-(1-S-Carbethoxy-3-cyclohexyl-propyl)-S-alanyl-spiro-[bicyclo[2.2.1]heptan-2,3'-pyrrolidin]5'-S-carbonsäure

N-(1-S-Carbethoxy-3-cyclohexyl-propyl)-S-lysyl-spiro-[bicyclo[2.2.1]heptan-2,3'-pyrrolidin]-5'-S-carbonsäure

N-(1-S-Carbethoxy-3-phenyl-propyl)-S-alanyl-spiro-[bicyclo[2.2.2]octan-2,3'-pyrrolidin]-5'-S-carbonsäure

N-(1-S-Carbethoxy-3-phenyl-propyl)-O-ethyl-tyrosyl-spiro-[bicyclo[2.2.2]octan-2,3'-pyrrolidin]-5'-S-carbonsäure

N-(1-S-Carbethoxy-3-phenyl-propyl)-S-lysyl-spiro[bicyclo-[2.2.2]octan-2,3'-pyrrolidin]-5'-S-carbonsäure

N-(1-S-Carbethoxy-3-cyclohexyl-propyl)-S-alanyl-spiro-[bicyclo[2.2.2]octan-2,3'-pyrrolidin]-5'-S-carbonsäure

N-(1-S-Carbethoxy-3-phenyl-propyl)-S-alanyl-2-azatricyclo-[4,3,0,$1^{6,9}$]decan-3-S-carbonsäure

N-(1-S-Carbethoxy-3-phenyl-propyl)-O-ethyl-S-tyrosyl-2-azatricyclo[4,3,0,$1^{6,9}$]decan-3-S-carbonsäure

N-(1-S-Carbethoxy-3-phenyl-propyl)-S-lysyl-2-azatricyclo-[4,3,0,$1^{6,9}$]decan-3-S-carbonsäure

N-(1-S-Carbethoxy-3-cyclohexyl-propyl)-S-alanyl-2-azatricyclo[4,3,0,$1^{6,9}$]decan-3-S-carbonsäure

N-(1-S-Carbethoxy-3-cyclohexyl-propyl)-S-lysyl-2-azatricyclo-[4,3,0,$1^{6,9}$]decan-3-S-carbonsäure

N-(1-S-Carbethoxy-3-phenyl-propyl)-S-alanyl-decahydrocyclohepta[b]pyrrol-2-S-carbonsäure

N-(1-S-Carbethoxy-3-phenyl-propyl)-O-ethyl-S-tyrosyl-decahydrocyclophepta[b]pyrrol-2-S-carbonsäure

N-(1-S-Carbethoxy-3-phenyl-propyl)-S-lysyl-decahydrocyclohepta[b]pyrrol-2-S-carbonsäure

N-(1-S-Carbethoxy-3-cyclohexyl-propyl)-S-alanyl-decahydrocyclohepta[b]pyrrol-2-S-carbonsäure

N-(1-S-Carbethoxy-3-cyclohexyl-propyl)-S-lysyl-decahydrocyclohepta[b]pyrrol-2-S-carbonsäure

N-(1-S-Carbethoxy-3-phenyl-propyl)-S-alanyl-trans-octahydroisoindol-1-S-carbonsäure

N-(1-S-Carbethoxy-3-phenyl-propyl)-S-alanyl-cis-octahydroisoindol-1-S-carbonsäure

N-(1-S-Carbethoxy-3-cyclohexyl-propyl)-S-alanyl-trans-octahydroisoindol-1-S-carbonsäure

N-(1-S-Carbethoxy-3-cyclohexyl-propyl)-S-alanyl-cis-octahydroisoindol-1-S-carbonsäure

N-(1-S-Carbethoxy-3-phenyl-propyl)-S-alanyl-cis-octahydrocyclopenta[c]pyrrol-1-S-carbonsäure

N-(1-S-Carbethoxy-3-cyclohexyl-propyl)-S-alanyl-cis-octahydrocyclopenta[c]pyrrol-1-S-carbonsäure

N-(1-S-Carbethoxy-3-cyclohexyl-propyl)-S-lysyl-cis-octahydrocyclopenta[c]pyrrol-1-S-carbonsäure

N-(1-S-Carbethoxy-3-phenyl-propyl)-S-alanyl-2,3,3a,4,5,7a-hexahydroindol-cis-endo-2-S-carbonsäure

N-(1-S-Carbethoxy-3-phenyl-propyl)-S-lysyl-2,3,3a,4,5,7a-hexahydroindol-cis-endo-2-S-carbonsäure

N-(1-S-Carbethoxy-3-cyclohexyl-propyl)-S-lysyl-2,3,3a,4,5,7a-hexahydroindol-cis-endo-2-S-carbonsäure

N-(1-S-Carbethoxy-3-phenyl-propyl)-S-alanyl-2,3,3a,4,5,7a-hexahydroindol-cis-exo-2-S-carbonsäure

N-(1-S-Carbethoxy-3-cyclohexyl-propyl)-O-ethyl-S-tyrosyl-2,3,3a,4,5,7a-hexahydroindol-cis-exo-2-S-carbonsäure

N-(1-S-Carbethoxy-3-cyclohexyl-propyl)-S-alanyl-2,3,3a,4,5,7-hexahydroindol-cis-endo-2-S-carbonsäure

N-(1-S-Carbethoxy-3-cyclohexyl-propyl) -lysyl-2-azabicyclo[3.1.0]hexan-3-S-carbonsäure

N-(1-S-Carbethoxy-3-phenyl-propyl) -lysyl-2-azabicyclo-[3.1.0]hexan-cis-endo-3-S-carbonsäure

N-(1-S-Carbethoxy-3-cyclopentylpropyl)-S-alanyl-2-aza-[3.1.0]hexan-3-carbonsäure

N-(1-S-Carbethoxy-3-phenyl-propyl)-S-alanyl-cis-endo-2-azabicyclo[3.1.0]hexan-3-S-carbonsäure

N-(1-S-Carbethoxy-3-cyclohexyl-propyl)-S-alanyl-cis-endo-2-azabicyclo[3.1.0]hexan-3-S-carbonsäure

und die entsprechenden Dicarbonsäuren der oben aufgeführten Verbindungen wie z.B.

N-(1-S-Carboxy-3-phenylpropyl)-S-alanyl-cis-endo-2-azabicyclo[3.3.0]octan-3-S-carbonsäure

N-(1-S-Carboxy-3-phenyl-propyl)-S-alanyl-S-1,2,3,4-tetrahydroisochinolin-3-carbonsäure etc.

Diese Verbindungen lassen sich nach dem in der deutschen Patentanmeldung P 33 33 455.2 beschriebenen Verfahren herherstellen, in dem die in der Anmeldung beschriebenen tert. Butyl- oder Benzylrest in bekannter Weise durch saure oder alkalische Hydrolyse oder durch Edelmetall-katalysierte Hydrogenolyse in die Monocarbonsäurederivate überführt werden. Die $N_2$-Benzyloxycarbonylschutzgruppe der Lysinderivate wird durch Edelmetall-katalysierte Hydrogenolyse entfernt. Die oben aufgeführten Verbindungen lassen sich

leicht mit physiologisch verträglichen Säuren oder Basen (im Falle von Mono- oder Dicarbonsäuren) in die entsprechenden Salze (z.B. Hydrochloride, Maleinate, Fumerate, etc.) überführen und als Salze die erfindungsgemäße Verwendung finden.

Die Verbindungen der Formel I sind Hemmstoffe des Angiotensin Converting Enzymes (ACE) beziehungsweise Zwischenprodukte bei der Herstellung von solchen Hemmstoffen und können auch zur Bekämpfung des Bluthochdrucks verschiedener Genese eingesetzt werden. Die Verbindungen der Formel I sind bekannt aus DE-OS 3211397, DE-OS 3227055, E-PA-46953, E-PA-79022, E-PA-84164, E-PA89637, E-PA-90362. Sie sind weiterhin Gegenstand der deutschen Patentanmeldungen P 32 42 151.6, P 32 46 503.3, P 32 46 757.5, P 33 00 774.8 und P 33 24 263.1.

In Ausübung der erfindungsgemäßen Methode können die oben beschriebenen Angiotensin Converting Enzyme-Inhibitoren an Säugern wie Affen, Hunden, Katzen, Ratten, Menschen etc. angewendet werden. Die für die erfindungsgemäße Verwendung geeigneten Verbindungen werden zweckmäßig in üblicher Weise in pharmazeutische Präparate eingearbeitet. Sie können in die üblichen Verabreichungsformen, wie Kapseln, Tabletten, Dragees, Lösungen, Salben, Emulsionen und auch in Depot-Form gebracht werden. Der Wirkstoff kann gegebenenfalls auch in mikroverkapselter Form vorliegen. Die Präparate können verträgliche, organische bzw. anorganische Begleitstoffe, beispielsweise Granulierstoffe, Klebe- und Bindemittel, Gleitmittel, Suspendiermittel, Lösungsmittel, antibakterielle Mittel, Netzmittel und Konservierungsmittel enthalten. Orale und topische Anwendungsformen werden bevorzugt. Auch parenterale Zubereitungen können angewendet werden. Bezüglich systemischer Anwendungsformen werden etwa 0,1 - 50 mg pro Dosis ein- bis dreimal täglich verabreicht. Für die topische Anwendung werden vorzugsweise Lösungen, Salben

oder ophthalmische inserts ("solid inserts") benutzt. Topische Anwendungsformen können 0,001 - 5 Gewichtsprozente des ACE-Inhibitors enthalten. Höhere oder niedrigere Dosierungen können ebenfalls angewendet werden, vorausgesetzt sie erniedrigen den Augeninnendruck. Vorzugsweise werden am menschlichen Auge 0,0001 - 5 mg bzw. 0,0005 - 1,0 mg und speziell 0,001 - 1,0 mg des ACE-Inhibitors angewendet. Für die bevorzugte topische Anwendung am Auge werden die ACE-Inhibitoren der Formel I in Kombination mit physiologisch verträglichen Trägerstoffen, wie z.B. wäßrige Methylcellulose appliziert. Die Kombination kann in Form einer Suspension, Lösung, Salbe, Emulsion oder eines Okusert bestehen. Eine bevorzugte Kombination ist die, die die Penetration der ACE-Inhibitoren in das Auge erleichtert. Eine weitere bevorzugte topische Anwendungsform ist die Kombination der ACE-Inhibitoren mit Verbindungen, wie z.B. Benzalkoniumchlorid, welches die Penetration des ACE-Inhibitors in das Auge erleichtert. Ebenso sind Prodrugs der ACE-Inhibitoren geeignet, wenn sie in der Cornea zur aktiven Verbindung hydrolysiert werden. ACE-Inhibitoren der Formel I können auch in Kombination mit anderen Antiglaucoma-Verbindungen zur Behandlung der Glaukomkrankheit eingesetzt werden.

Die topischen Trägerstoffe können organische oder anorganische Verbindungen sein. Typische pharmazeutische gebrauchte Trägerstoffe sind wäßrige Lösungen, die z.B. Puffersysteme oder isotonische Mischungen von Wasser und wassermischbaren Lösungsmitteln sind, wie z.B. Alkohole oder Arylalkohole, Öle, Polyalkylenglykole, Ethylcellulose, Carboxymethylcellulose, Polyvinylpyrrolidon oder Isopropylmyristat. Geeignete Puffersubstanzen sind z.B. Natriumchlorid, Natriumborat, Natriumphosphat, Natriumacetat oder auch Gluconatpuffer. Die topische Anwendungsform kann auch nicht toxische Hilfsstoffe enthalten wie

z.B. emulgierende Konservierungsstoffe, Vernetzer, wie z.B. Polyethylenglykole, antibakterielle Verbindungen, wie z.B. quarternäre Ammoniumverbindungen, Benzalkoniumchlorid, Phenylquecksilbersalze, Benzylalkohol, Phenylethanol, Triethanolaminoleate, Thiosorbitol und weitere ähnliche Stoffe, die in topischen ophthalmischen Zubereitungen gebraucht werden. Die topische Anwendungsform kann ebenso in Form eines ophthalmischen inserts ("solid insert") bestehen. Hierzu kann z.B. ein festes wasserlösliches Polymer als Carrier für den ACE-Inhibitor gebraucht werden. Als Polymer kann jedes wasserlösliche, nicht toxische Polymer benutzt werden, wie z.B. Cellulosederivate, wie z.B. Methylcellulose, Natriumcarboxymethylcellulose, $C_{1-6}$ -Hydroxyalkylcellulose, wie z.B. Hydroxyethylcellulose, Hydroxypropylcellulose und Hydroxypropylmethylcellulose, Acrylsäurederivate, wie Polyacrylsäuresalze, Äthylacrylate und Polyacrylamide können gebraucht werden. Weiterhin können z.B. Gelatine, Stärkederivate, Alginate, Pektine, Polyvinylalkohole, Polyvinylpyrrolidone, Polyvinylmethylether, Polyethylenoxide oder auch auch Mischungen von den verschiedenen Polymeren benutzt werden. Eine Beschreibung dieser solid inserts, die für eine topische Anwendung in Frage kommen, gibt die britische Patentanmeldung 1524405.

Die folgenden Beispiele geben die Anwendungsformen zur Behandlung des Glaukoms nach der erfindungsgemäßen Methode an. Die Verbindungen der Formel I können analog den Beispielen in die entsprechenden Anwendungsformen gebracht werden.

Beispiel 1

Herstellung des erfindungsgemäß verwendeten Mittels zur oralen Anwendung in der Behandlung des Glaukoms.

1000 Tabletten, die je 10 mg 1-N-(1-S-Carbethoxy-3-phenyl-propyl)-S-alanyl-1S,3S,5S-2-azabicyclo[3.3.0]-octan-3-carbonsäure enthalten, werden mit den folgenden Hilfsmitteln hergestellt:

| | |
|---|---|
| N-(1-S-Carbethoxy-3-phenyl-propyl)-S-alanyl-1S,3S,5S-2-azabicyclo[3.3.0]octan-3-carbonsäure | 10 g |
| Maisstärke | 140 g |
| Gelatine | 7,5 g |
| Mikrokristalline Cellulose | 2,5 g |
| Magnesiumstearat | 2,5 g |

N-(1-S-Carbethoxy-3-phenyl-propyl)-S-alanyl-1S,3S,5S,-2-azabicyclo[3.3.0]octan-3-carbonsäure und Maisstärke werden mit einer wäßrigen Gelatine-Lösung gemischt. Die Mischung wird getrocknet und zu einem Granulat vermahlen. Mikrokristalline Cellulose und Magnesiumstearat werden mit dem Granulat vermischt. Das entstandene Granulat wird zu 1000 Tabletten gepreßt, wobei jede Tablette 10 mg des ACE-Hemmers enthält.

Diese Tabletten können zur Behandlung des Glaukoms oder zur Herabsetzung des Augeninnendrucks benutzt werden.

Beispiel 2

Analog Beispiel 1 werden 1000 Tabletten hergestellt, die je 10 mg N-(1-S-Carbethoxy-3-phenyl-propyl)-S-alanyl-(2S,3aR,7aS)-octahydroindol-2-carbonsäure-hydrochlorid enthalten.

Beispiel 3

Gelatine-Kapseln, die je 10 mg N-(1-S-Carbethoxy-3-phenyl-propyl)-S-alanyl-1S,3S,5S-2-azabicyclo[3.3.0]octan-3-carbonsäure enthalten, werden mit der folgenden Mischung gefüllt:

| | |
|---|---|
| N-(1-S-Carbethoxy-3-phenyl-propyl)-S-alanyl-1S,3S,5S-2-azabicyclo[3.3.0]octan-3-carbonsäure | 10 mg |
| Magnesiumstearat | 1 mg |
| Lactose | 214 mg |

Diese Kapseln können zur Behandlung des Glaukoms oder zur Herabsetzung des Augeninnendrucks benutzt werden.

Beispiel 4

Die Herstellung einer Injektionslösung zur Behandlung des Glaukoms wird im folgenden beschrieben:

| | |
|---|---|
| N-(1-S-Carboxy-3-phenyl-propyl)-S-alanyl-1S,3S,5S-2-azabicyclo[3.3.0]octan-3-carbonsäure | 250 mg |
| Methylparaben | 5 g |
| Propylparaben | 1 g |
| Natriumchlorid | 25 g |
| Wasser für Injektion | 5 l |

N-(1-S-Carboxy-3-phenyl-propyl)-S-alanyl-1S,3S,5S-2-azabicyclo[3.3.0]octan-3-carbonsäure, die Konservierungsstoffe und Natriumchlorid werden in 3 l Wasser für Injektion gelöst und auf 5 l mit Wasser für Injektion aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

Beispiel 5

Die Herstellung einer sterilen Lösung für die topische Anwendung am Auge zur Behandlung des Glaukoms oder Herabsetzung des Augeninnendrucks wird im folgenden beschrieben:

| | | |
|---|---|---|
| N-(1-S-Carbethoxy-3-phenyl-propyl)-S-alanyl-1S,3S,5S-2-azabicyclo[3.3.0]octan-3-carbonsäure | 1 mg | 10 mg |

| | | |
|---|---|---|
| Natriumphosphat , monobasisch x $H_2O$ | 9,4 mg | 4,1 mg |
| Dibasisches Natriumphosphat x 12 $H_2O$ | 28,5 mg | 11,2 mg |
| Benzalkoniumchlorid | 0,1 mg | 0,1 mg |
| Natriumhydroxidlösung zur Einstellung auf | pH 6,8 | pH 6,8 |
| Wassser zur Injektion | 10 ml | 10 ml |

Der ACE-Inhibitor, die Phosphat-Salze und Benzalkonium-chlorid werden in Wasser gelöst, mit wäßriger Natriumhydroxidlösung auf pH 6,8 gebracht und mit Wasser zum gewünschten Volumen aufgefüllt. Die Lösung wird steril filtriert.

Beispiel 6

Eine sterile Lösung für die topische Anwendung am Auge zur Behandlung des Glaukoms oder zur Herabsetzung des Augeninnendrucks wird wie in Beispiel 5 beschrieben hergestellt mit der Ausnahme, daß anstelle von N-(1-S-Carbethoxy-3-phenyl-propyl)-S-alanyl-1S,3S,5S-2-azabicyclo[3.3.0]octan-3-carbonsäure, N-(1-S-Carboxy-3-phenyl-propyl)-S-alanyl-1S,3S,5S-2-azabicyclo[3.3.0]octan-3-carbonsäure oder N-(1-S-Carbethoxy-3-phenyl-propyl)-S-alanyl-2S,3aR,7aS-octahydroindol-2-carbonsäurehydrochlorid oder N-(1-S-Carboxy-3-phenyl-propyl)-S-alanyl-2S,3aR,7aS-octahydroindol-2-carbonsäure oder N-(1-S-Carbethoxy-3-phenyl-propyl)-S-alanyl-cis-2,3,3a,4,5,7a-hexahydro[1H]indol-2-S-endo-carbonsäure oder N-(1-S-Carboxy-3-phenyl-propyl)-S-alanyl-cis-2,3,3a,4,5,7a-hexahydro[1H]indol-2-S-endo-carbonsäure oder N-(1-S-Carboxy-3-phenyl-propyl)-S-lysyl-1S,3S,5S-2-azabicyclo-[3.3.0]octan-3-carbonsäure oder N-(1-S-Carbethoxy-3-cyclohexyl-propyl)-S-alanyl-1S,3S,5S-2-azabicyclo-[3.3.0]octan-3-carbonsäure oder N-(1-S-Carboxy-3-cyclohexyl-propyl)-S-lysyl-1S,3S,5S-2-azabicyclo-[3.3.0]octan-3-carbonsäure oder N-(1-S-Carboxy-3-phenyl-propyl)-S-alanyl-S1,2,3,4-tetrahydroisochinolin-3-S-carbonsäure angewendet werden.

Beispiel 7

Eine sterile Lösung für die topische Anwendung am Auge zur Behandlung des Glaukoms oder zur Herabsetzung des Augeninnendrucks wird wie folgt hergestellt:

| | |
|---|---|
| N-(1-S-Carboxy-3-phenyl-propyl)-S-lysyl-1S,3S,5S-2-azabicyclo[3.3.0]octan-3-carbonsäure | 1 mg/ml |
| Ethylendiamintetraessigsäure-dinatriumsalz-dihydrat | 0,4 mg/ml |
| Natriumchlorid | 7,4 mg/ml |
| Natronlauge, Salzsäure | pH 4,5 |
| Wasser zur Injektion | 1,0 ml |

Wasser zur Injektion wird vorgelegt. Dazu werden zuerst Ethylendiamintetraessigsäuredinatriumsalz-dihydrat und Natriumchlorid zugegeben und darauf der ACE-Inhibitor. Der pH wird mit Natronlauge bzw. Salzsäure auf pH 4,5 eingestellt. Es wird dann weiteres Wasser zur Injektion zugegeben, um die Lösung zum gebrauchten Volumen zu bringen.

Beispiel 8

Ophthalmische "inserts" (geformte Arzneizubereitungen die zum Einlegen in den Bindehautsack oder die zum Auflegen auf den Augapfel bestimmt sind) für die Anwendung zur Behandlung des Glaukoms oder zur Herabsetzung des Augeninnendrucks können wie folgt hergestellt werden:

| | |
|---|---|
| N-(1-S-Carbethoxy-3-phenyl-propyl)-S-alanyl-1S,3S,5S-2-azabicyclo[3.3.0]octan-3-carbonsäure | 1 mg |
| Hydroxypropylcellulose | 12 mg |

Der ACE-Inhibitor und die Hydroxypropylcellulose werden mit Methanol zu einer viskosen Lösung verarbeitet. Diese

Lösung wird auf einer Polytetrafluorethylen-Platte angestrichen. Den Film läßt man bei Raumtemperatur trocknen. Nach dem Trocknen wird der Film in einer Kammer mit hoher Luftfeuchte geschmeidig gemacht. Der Film wird dann in der zur Anwendung geeignete Stücke geschnitten.

Beispiel 9

Ophthalmische "inserts" für die Anwendung am Auge zur Behandlung des Glaukoms, oder zur Herabsetzung des Augeninnendrucks werden wie in Beispiel 8 beschrieben hergestellt mit der Ausnahme, daß anstelle von N-(1-S-Carbethoxy-3-phenyl-propyl)-S-alanyl-1S,3S,5S-2-azabicyclo-[3.3.0]octan-3-carbonsäure, N-(1-S-Carboxy-3-phenyl-propyl)-S-alanyl-1S,3S,5S-2-azabicyclo[3.3.0]octan-3-carbonsäure oder N-(1-S-Carbethoxy-3-phenyl-propyl)-S-alanyl-2S,3aR,7aS-octahydroindol-2-carbonsäurehydrochlorid oder N-(1-S-Carboxy-3-phenyl-propyl)-S-alanyl-2S,3aR,7aS-octahydroindol-2-carbonsäure oder N-(1-S-Carbethoxy-3-phenyl-propyl)-S-alanyl-cis-2,3,3a,4,5,7a-hexahydro[1H]indol-2-S-endo-carbonsäure oder N-(1-S-Carboxy-3-phenyl-propyl)-S-alanyl-cis-2,3,3a,4,5,7a-hexyhydro[1H]indol-2-S-endo-carbonsäure oder N-(1-S-Carboxy-3-phenyl-propyl)-S-lysyl-1S,3S,5S-2-azabicyclo-[3.3.0]octan-3-carbonsäure oder N-(1-S-Carbethoxy-3-cyclohexyl-propyl)-S-alanyl-1S,3S,5S-2-azabicyclo-[3.3.0]octan-3-carbonsäure oder N-(1-S-Carboxy-3-cyclohexyl-propyl)-S-lysyl-1S,3S,5S-2-azabicyclo-[3.3.0]octan-3-carbonsäure oder N-(1-S-Carboxy-3-phenyl-propyl)-S-alanyl-S-1,2,3,4-tetrahydroisochinolin-3-carbonsäure angewendet werden.

<u>Patentansprüche:</u>

1. Methode zur Behandlung des Glaukoms und/oder der Herabsetzung des Augeninnendrucks in Säugern durch Anwendung von Angiotensin-Converting-Enzyme-Inhibitoren der Formel I

$$R^3OOC - \underset{R^4}{\overset{*}{CH}} - \underset{R^5}{N} - \underset{O}{\overset{\|}{C}} - \underset{R^1}{\overset{*}{CH}} - NH - \underset{COOR^2}{\overset{*}{CH}} - (CH_2)_n-R \qquad (I)$$

in welcher

n= 1 oder 2 ist,

R= Wasserstoff,
einen gegebenenfalls substituierten aliphatischen Rest mit 1 - 8 C-Atomen,
einen gegebenenfalls substituierten alicyclischen Rest mit 3 - 9 C-Atomen,
einen gegebenenfalls substituierten aromatischen Rest mit 6 - 12 C-Atomen,
einen gegebenenfalls substituierten araliphatischen Rest mit 7 - 14 C-Atomen,
einen gegebenenfalls substituierten alicyclisch-aliphatischen Rest mit 7 - 14 C-Atomen,
einen Rest $OR^a$ oder $SR^a$, worin

$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,

$R^1$ Wasserstoff,
einen gegebenenfalls substituierten aliphatischen Rest mit 1 - 6 C-Atomen,
einen gegebenenfalls substituierten alicyclischen Rest mit 3 - 9 C-Atomen,

einen gegebenenfalls substituierten alicyclisch-aliphatischen Rest mit 4 - 13 C-Atomen, einen gegebenenfalls substituierten aromatischen Rest mit 6 - 12 C-Atomen, einen gegebenenfalls substituierten araliphatischen Rest mit 7 - 16 C-Atomen, einen gegebenenfalls substituierten heteroaromatischen Rest mit 5 - 12 Ringatomen oder die erforderlichenfalls geschützte Seitenkette einer natürlich vorkommenden α-Aminosäure bedeuten,

$R^2$ und $R^3$ gleich oder verschieden sind und Wasserstoff, einen gegebenenfalls substituierten aliphatischen Rest mit 1 - 6 C-Atomen, einen gegebenenfalls substituierten alicyclischen Rest mit 3 - 9 C-Atomen, einen gegebenenfalls substituierten aromatischen Rest mit 6 - 12 C-Atomen, einen gegebenenfalls substituierten araliphatischen Rest mit 7 - 16 C-Atomen bedeuten und

$R^4$ und $R^5$ zusammen mit den sie tragenden Atomen ein mono-, bi-, oder tricyclisches heterocyclisches Ringsystem mit 5 bis 15 C-Atomen bilden sowie deren physiologisch verträglichen Salzen.

2. Methode gemäß Anspruch 1, dadurch gekennzeichnet, daß Verbindungen der Formel I angewendet werden, worin $R^4$ und $R^5$ zusammen mit dem sie tragenden Atomen für ein gegebenenfalls substituiertes System aus der Reihe Tetrahydroisochinolin, Decahydroisochinolin, Octahydroindol, Octahydrocyclopenta[b]pyrrol, 2-Aza-bicyclo[2.2.2]octan, 2-Azabicyclo[2.2.1]heptan, 2-Azaspiro[4.5]decan, 2-Azaspiro[4.4]nonan, Spiro[(bicyclo[2.2.1]heptan)-2,3-pyrrolidin], Spiro[(bicyclo-[2.2.2]octan)-2,3-pyrrolidin], 2-Azatricyclo[4,3,0,$1^{6,9}$]decan, Decahydrocyclohepta[b]pyrrol, Octahydroisoindol, Octahydrocyclopenta[c]pyrrol, 2,3,3a,4,5,7a-Hexahydroindol, 2-Azabicyclo[3.1.0]-hexan stehen.

3. Methode gemäß einem der Ansprüche 1 oder 2, dadurch gekennzeichnet, daß Verbindungen der Formel I angewendet werden, in welcher n = 1 oder 2 ist

R Wasserstoff mit 1 - 8 C-Atomen,
Alkenyl mit 2 - 6 C-Atomen,
Cycloalkyl mit 3 - 9 C-Atomen,
Aryl mit 6 - 12 C-Atomen,
das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-Alkylamino, Methylendioxy, Carboxy, Cyano und/oder Sulfamoyl mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel- oder Sauerstoffatome und/oder 1 bis 4 Ringatome Stickstoff darstellen,
das wie oben bei Aryl beschrieben substituiert sein kann,

Amino-$(C_1-C_4)$-alkyl,
$(C_1-C_4)$-Alkanoylamino-$(C_1-C_4)$alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4$-Alkylthio-$(C_1-C_4)$-alkyl,
$(C_6-C_{12})$-Arylthio-$(C_1-C_4$-alkyl,

das im Arylteil wie oben bei Aryl beschrieben, substituiert sein kann,

$(C_6-C_{12})$-Aryl-$(C_1-C_4)$-alkylthio,

das im Arylteil wie oben bei Aryl beschrieben substituiert sein kann,

Carboxy-$(C_1-C_4)$-alkyl,

Carboxy, Carbamoyl,

Carbamoyl-$(C_1-C_4)$-alkyl,

$(C_1-C_4)$-Alkoxy-carbonyl-$(C_1-C_4)$-alkyl,

$(C_6-C_{12})$-Aryloxy-$(C_1-C_4)$-alkyl,

das im Arylteil wie oben bei Aryl beschrieben substituiert sein kann oder

$(C_6-C_{12})$-Aryl-$(C_1-C_4)$-alkoxy,

das im Arylteil wie oben bei Aryl beschrieben substituiert sein kann,

$R^1$ Wasserstoff,

Alkyl mit 1 - 6 C-Atomen,

Alkenyl mit 2 - 6 C-Atomen,

Alkinyl mit 2 - 6 C-Atomen,

Cycloalkyl mit 3 - 9 C-Atomen,

Cycloalkenyl mit 5 - 9 C-Atomen,

$(C_3-C_9)$-Cycloalkyl-$(C_1-C_4)$-alkyl,

$(C_5-C_9)$-Cycloalkenyl-$(C_1-C_4)$-alkyl,

gegebenenfalls teilhydriertes Aryl mit 6 - 12 C-Atomen, das wie oben bei R beschrieben substituiert sein kann,

$(C_6-C_{12})$ -Aryl-$(C_1-C_4)$-alkyl oder $(C_7-C_{13})$-Aroyl-$(C_1$ oder $C_2)$ alkyl

die beide wie das vorstehende Aryl substituiert sein können

mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,

das wie das vorstehende Aryl substituiert sein kann oder

die gegebenenfalls geschützte Seitenkette einer natürlich vorkommenden α-Aminosäure $R^1$-CH($NH_2$)-COOH bedeuten,

$R^2$ und $R^3$ gleich oder verschieden sind und
Wasserstoff,
Alkyl mit 1 - 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,
($C_3$-$C_9$)-Cycloalkyl oder
($C_3$-$C_9$)-Cycloalkyl-($C_1$-$C_4$)-alkyl
bedeuten und

$R^4$ und $R^5$ die oben angegebene Bedeutung haben.

4. Methode gemäß einem der Ansprüche 1 bis 3, dadurch gekennzeichnet, daß [S,S,S,S,S]-N-(1-Carbethoxy-3-phenyl-propyl)-alanyl-octahydroindol-2-carbonsäure angewendet wird.

5. Methode gemäß einem der Ansprüche 1 bis 3, dadurch gekennzeichnet, daß N-(1-(S)-Carbethoxy-3-phenyl-propyl)-(S)-alanyl-3aR,7aS-octahydroindol-2-(S)-carbonsäure angewendet wird.

6. Methode gemäß einem der Ansprüche 1 bis 3, dadurch gekennzeichnet, daß [S,S,S,S,S]-N-(1-Carbethoxy-3-phenyl-propyl)-alanyl-decahydroisochinolin-3-carbonsäure angewendet wird.

7. Methode gemäß einem der Ansprüche 1 bis 3, dadurch gekennzeichnet, daß [S,S,S]-N-(1-Carbethoxy-3-phenyl-propyl)-alanyl-tetrahydroisochinolin-3-carbonsäure angewendet werden.

8. Methode gemäß einem der Ansprüche 1 bis 3, dadurch gekennzeichnet, daß (S,S,S,S,S)-N-(1-Carbethoxy-3-phenyl-propyl)-alanyl-2-azabicyclo[3.3.0]octan-3-carbonsäure angewendet wird.

9. Methode gemäß einem der Ansprüche 1 bis 3, dadurch gekennzeichnet, daß N-(1-S-Carbethoxy-3-phenyl-propyl)-S-alanyl-cis-endo-2-azabicyclo[3.1.0]hexan-3-S-carbonsäure angewendet wird.

10. Methode gemäß einem der Ansprüche 1 bis 3, dadurch gekennzeichnet, daß N-(1-S-Carbethoxy-3-phenyl-propyl)-S-alanyl-cis-endo-2,3,3a,4,5,7a-hexahydroindol-2-S-carbonsäure angewendet wird.

11. Methode gemäß einem der Ansprüche 4 bis 10, dadurch gekennzeichnet, daß anstelle der Ethylester die entsprechenden Dicarbonsäuren angewendet werden.

12. Methode gemäß einem der Ansprüche 1 bis 3 oder 11, dadurch gekennzeichnet, daß N-(1-S-Carboxy-3-phenyl-propyl)-S-alanyl-S-1,2,3,4-tetrahydroisochinolin-3-carbonsäure angewendet wird.

13. Methode gemäß Anspruch 1, dadurch gekennzeichnet, daß die anwendungsgemäßen Angiotensin-Converting-Enzyme-Inhibitoren oral oder parenteral angewendet werden.

14. Methode gemäß Anspruch 1, dadurch gekennzeichnet, daß die anwendungsgemäßen Angiotensin-Converting-Enzyme-Inhibitoren topisch mittels Lösungen oder ophthalmischen Inserts angewendet werden.

15. Methode gemäß Anspruch 1, dadurch gekennzeichnet, daß die anwendungsgemäßen Angiotensin-Converting-Enzyme-Inhibitoren für die entsprechenden Anwendungsformen mit pharmazeutisch geeigneten Trägerstoffen bzw. Hilfsstoffen kombiniert werden.

16. Verbindung der Formel I gemäß einem der Ansprüche 1 bis 12, sowie deren physiologisch verträgliche Salze zur Anwendung als Mittel zur Behandlung des Glaukoms und/oder zur Herabsetzung des Augeninnendrucks.

17. Pharmazeutisches Mittel enthaltend eine Verbindung der Formel I gemäß einem der Ansprüche 1 bis 12 oder deren physiologisch verträgliches Salz zur Anwendung bei der Behandlung des Glaukoms und/oder zur Herabsetzung des Augeninnendrucks bei Säugern.

18. Verwendung einer Verbindung der Formel I gemäß einem der Ansprüche 1 bis 12 oder eines Mittels gemäß Anspruch 17 zur Behandlung des Glaukoms und/oder zur Herabsetzung des Augeninnendrucks bei Säugern.

0158157

Nummer der Anmeldung

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

EP 85103022.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2 - 0 099 239 (E.R. SQUIBB & SONS, INC.)<br>* Ansprüche; Seiten 13-16 *<br>-- | 16,17 | A 61 K 31/40<br>A 61 K 31/435<br>C 07 D 209/52<br>C 07 D 209/54<br>C 07 D 209/42 |
| P,A | EP - A2 - 0 114 333 (SCHERING CORPORATION)<br>* Ansprüche; Seiten 8-10 *<br>-- | 16,17 | |
| D,X | DE - A1 - 3 211 397 (HOECHST AG)<br>* Ansprüche *<br>-- | 16,17 | |
| D,X | DE - A1 - 3 227 055 (HOECHST AG)<br>* Ansprüche *<br>-- | 16,17 | |
| D,X | EP - A2/A3 - 0 079 022 (HOECHST AG)<br>* Ansprüche *<br>-- | 16,17 | |

**RECHERCHIERTE SACHGEBIETE (Int. Cl.4)**

A 61 K 31/00
A 61 K 45/00
A 61 K 37/00
C 07 D 209/00
C 07 D 453/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 16,17
Unvollständig recherchierte Patentansprüche: -
Nicht recherchierte Patentansprüche: 1-15,18
Grund für die Beschränkung der Recherche:

(Verfahren zur therapeutischen Behandlung des menschlichen oder tierischen Körpers, Art. 52(4) EPÜ)

| Recherchenort | Abschlußdatum der Recherche | Prüfer |
|---|---|---|
| WIEN | 28-06-1985 | STÖCKLMAYER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1505.1 08.82

0158157

Europäisches Patentamt

# EUROPÄISCHER TEILRECHERCHENBERICHT

Nummer der Anmeldung

EP 85103022.1

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| D,X | EP - A2 - 0 090 362 (HOECHST AG)<br>* Ansprüche *<br>-- | 16,17 | |
| D,X | EP - A2 - 0 089 637 (HOECHST AG)<br>* Ansprüche *<br>-- | 16,17 | |
| D,X | EP - A2/A3 - 0 084 164 (HOECHST AG)<br>* Ansprüche *<br>-- | 16,17 | RECHERCHIERTE SACHGEBIETE (Int. Cl. 4) |
| D,X | EP - A2 - 0 046 953 (HOECHST AG)<br>* Ansprüche *<br>---- | 16,17 | |